# EUROPEAN PATENT SPECIFICATION

(11) **EP 1 779 798 B1**
(45) Date of publication and mention of the grant of the patent: **18.02.2009**
(21) Application number: 05023270.1
(22) Date of filing: 25.10.2005
(51) Int. Cl.: A61B 17/88

(54) **Non-penetrating fixing device**
Nichteindringende Befestigungsvorrichtung
Dispositif de fixation non-pénétrant

(43) Date of publication of application: 02.05.2007
(73) Proprietor: BrainLAB AG, 85622 Feldkirchen (DE)
(72) Inventor: Maier, Georg, 81541 Munich (DE); Maier, Christian, 80802 Munich (DE)
(74) Representative: Schwabe - Sandmair - Marx

(56) References cited:
- DE-U1- 20 015 893
- US-A- 4 009 712
- US-A- 5 709 682
- US-A- 5 945 548
- US-A1- 2001 053 911
- US-A1- 2005 149 050

## Description

The present invention relates to a device for securely fixing surgical equipment to a surgical field of a human or animal body, in particular a bone, according to claim 1.

Modem surgical procedures require an improved accuracy to guarantee a minimally invasive operation, such that the patient is able to recover from any surgery. Therefore, it is necessary to use a surgical procedure which incurs a minimal injury to the patient's body. To be able to avoid injuring the patient's body during a surgical operation, it is necessary to operate on the patient to an improved accuracy, while a minimally invasive procedure does not allow the surgeon a direct view in order of to monitor the surgical field. In this connection, it is nowadays usual to use a reference element or reference array to allow a surgeon some points of orientation in the surgical field. To maintain the accuracy and the minimally invasive surgical technique, it is very important to guarantee that the reference element or reference array cannot be shifted, rotated or misplaced during surgery. Misplacing the reference element or reference array would mislead the surgeon away from the actual situation in the surgical field and would cause surgical errors.

Several different attachment methods are described in the prior art. In general, there are two groups of methods in the prior art. One of these groups is directed to a bone attachment method according to which a bone is penetrated and a part of the fixing device is introduced into the bone. This group incurs the drawback that it is necessary to penetrate and thus injure a patient's body. In the other group, it is necessary to open a wide portal through the soft-issue of a patient, in order to introduce a fixing device which needs a lot of space in order to entered and be fixed to a patient's bone. A list of publications directed to these two groups of methods is listed below:
6,860,883 External fixation apparatus and method
6,835,197 Bone fixation system
6,830,571 Contourable spinal staple with centralized and unilateral prongs
6,790,234 Reverse shoulder prosthesis system
6,730,086 Repositioning instrument to fixate bone-fractures
6,689,134 Longitudinal plate assembly having an adjustable length
6, 685,708 Staples for bone fixation
6, 685, 706 Proximal anchors for bone fixation system
6, 632,224 Bone fixation system
6,540,770 Reversible fixation device for securing an implant in bone
6,461,358 Device for the external fixation of bones fractures, in particular ankle fractures 6,447,517 Instrument for inserting graft fixation device
6,391,030 Surgical cable system and method
6,364,884 Method of securing a graft using a graft fixation device
6,328,758 Suture anchor with reversible expansion
6,206,826 Devices and methods for percutaneous surgery
6,179,840 Graft fixation device and method
6,102,912 Vertebral rod of constant section for spinal osteosynthesis instrumentations
5,827,286 Incrementally adjustable tibial osteotomy fixation device and method
5,674,221 External fixator with improved clamp and methods for use
5,569,303 Apparatus and method for attaching an object to bone
5,536,271 Patella reaming system
5,423,821 Sternal closure device
5,133,720 Surgical drill guide and retractor
5,026,372 Fixation device for the external adjusting of bone fragments
4,959,065 Bone plate with positioning member
4,944,743 Spinal Fixation device
4,655,776 Prostheses for ossicular reconstruction
4,587,916 Device for fixing the wish-bone of the sail-board to the mast in a fast and rigid manner
4,414,967 Internal fixation of bone, tendon, and ligaments
4,252,121 Separating device
3,987,500 Surgically implantable total ankle prosthesis

US-A-4,009,712 discloses pliers with a pair of crossed lever arms. The pliers are connected with each other through a pivot pin. The pin 54 permits rotation but obviates separation of the arms.

Accordingly, it is not possible to introduce the levers or arms separately and to withdraw the levers or arms separately from the field of surgery. The pliers is mounted before it is introduced into the field of surgery and it is only intended to dismantle the pliers after it has been withdrawn from the field of surgery.

The same technical teaching is derivable from SU 2001/053911 A1, DE 2001 5893 U1 and US-A-5,945,548.

It is the object of the present invention to provide a fixing device which allows a minimal invasive operating technique. It is another object of the present invention to provide a fixing device which can be fixed to a bone without penetrating and injuring the bone. Another object of the present invention is to provide a fixing device which allows the surgeon to use a small soft-issue portal in the patient in order to be able to insert the fixing device.

At least one of these objects is solved by a fixing device comprising the features as set forth in claim 1.

The advantages according to the present invention are achieved by means of a fixing device comprising a first lever element with a first gripping portion and a first handling portion, and a second lever element with a second gripping portion and a second handling portion, wherein said first and said second lever elements can be connected such that they can be pivoted and detached with respect to each other. Accordingly, one benefit of the present invention is that the fixing device according to the invention can be fixed to a patient's bone without penetrating the bone tissue. Furthermore, it is possible to connect and to detach elements of the fixing device according to the invention, such so that it is possible to introduce these elements into the patient's body through a comparatively small portal in the soft tissue of the patient's body.

While the intention is to fix a reference element or several reference elements or a reference array consisting of several reference elements to the fixing device, it is also possible to fix other surgical equipment to the fixing device according to the invention. For instance it can be possible to fix a probe, a lamp, an x-ray source or the like to the fixing device according to the present invention.

According to one advantageous embodiment of the present invention, one of the first and second lever elements comprises a receiving section and the other comprises an insertion section. The insertion section can be introduced into the receiving section such that the insertion section can be removed and is received in the receiving section such that it can be pivoted. This construction allows the fixing device according to the invention to be assembled in the surgical field in the patient's body, and the parts of the fixing device to be introduced and removed separately, the parts acting within the surgical field commonly having for instance the general shape of pliers.

According to another advantageous embodiment of the present invention, the fixing device comprises a securing element for securely maintaining an angle between the lever elements, in order to securely grip a bone. Accordingly, when a surgeon closes the lever elements of the device such that the bone is clamped between the gripping portions, this urging contact between the gripping elements and the bone can be maintained by means of the securing element, which can for instance be a screw or comparable component. This securing element can hold the levers together in their intended positions.

According to one embodiment of the invention, the receiving section has a hole-like, groove-like or slit-like shape for receiving the insertion section or for guiding the insertion section into an operating position within the receiving section. The insertion section can be held and pivoted or rotated in the receiving section, in order to fix the device according to the invention at a certain position on a bone.

According to another preferred embodiment, the device according to the invention comprises at least one gripping surface on each of the first and second gripping portions. Providing special gripping surfaces enables the surgeon to securely fix the device according to the invention on the bone. The gripping surfaces can have particular structures, for instance ridges, spikes or the like.

To allow the first lever element to pivot or rotate with respect to the second lever element of the device of the present invention, a guide surface is formed on the first or second lever element. The other of the first and second lever element is provided with a joint section having a sliding surface. The sliding surface co-operates with a guide surface, to allow the pivoting or rotating movement. The joint section is formed integrally with the other of the first and second lever elements.

According to another improved embodiment of the present invention, the gripping surface or gripping surfaces are broadened in a direction perpendicular to the main extension of the corresponding lever element and/or are provided with at least one, preferably two extended edges directed towards the opposite gripping surface. This allows a further safety aspect, in order to guarantee that the device of the invention cannot be displaced, rotated or the like, such that the reference elements or the reference array cannot be misplaced.

The fixing device according to any one of the embodiments of the invention can be introduced into a surgical field by means of the following steps:

Firstly, a soft tissue portal is opened in a patient's body. The first and second lever elements are then separately introduced, one after the other, through said portal. The first and second lever elements are then connected with each other. The first and second lever elements are then rotated or pivoted with respect to a common rotational centre, in order to pivot the first and second gripping portions towards each other and towards the surface of a bone to be gripped by the device, i.e. to be clamped between the gripping surfaces of the first and second lever elements.

It can be beneficial to ensure that the device according to the invention is fixed to a bone such that at least three portions or three areas of the device are in direct contact with corresponding parts of the bone. This prevents the device according to the invention from being rotated, once it has been fixed to the bone.

Accordingly, the present invention enables a surgeon to attach a device and in particular a reference element or reference array to a surgical field, and in particular to a bone structure of a patient. Reference element or reference arrays are commonly used for image-guided surgery. The device of the invention prevents translational and rotational displacements of the reference element or reference array or of any other surgical equipment which is fixed by means of the device according to the invention. The surgical tool according to the invention can be used in particular in connection with computer aided and/or image guided surgical systems like Vector Vision and/or Trauma 2.5, both well known products of the applicant.

Since the device according to the invention does not need to be fixed by penetrating a bone, the device according to the invention does not block the volume of the bone, preventing the insertion of implants.

As stated above, the fixing device according to the invention preferably comprises two levers. These levers can be formed such that the outer cortex of the bone fits the inner shape of the closed gripping surfaces of the levers. The levers are only assembled to form a plier-like surgical tool once the levers have been inserted separately through a soft tissue portal in the patient. Accordingly, it is a very important feature of the invention that the lever elements can be assembled by means of a combining element or joint element to allow the levers to be connected and to pivot or rotate, once introduced into the surgical field in order to form the plier-like instrument. For instance, a plug and a corresponding hole can be used to mount the lever elements. On the other hand, it is also possible to use a more complicated joint or combining element, comprising for instance a bolt which can be introduced into a bearing or the like, such that it can be rotated or pivoted. A hinge-like shape could also be used.

A brief description of the drawings illustrating some preferred embodiments of the invention, is given below, wherein the drawings show:
- Figure 1: a side view of the device according to one embodiment of the invention, wherein one of the levers is shown in a partially sectional view;
- Figure 1a: a sectional view along the line A-A in Figure 1;
- Figure 1b: a cross-section along the line B-B in Figure 1; and
- Figure 2: a specific embodiment of a securing element which can be used in connection with the embodiment shown in Figure 1.

In the drawings the same or comparable parts are designated by the same reference numbers, to avoid describing the same parts twice.

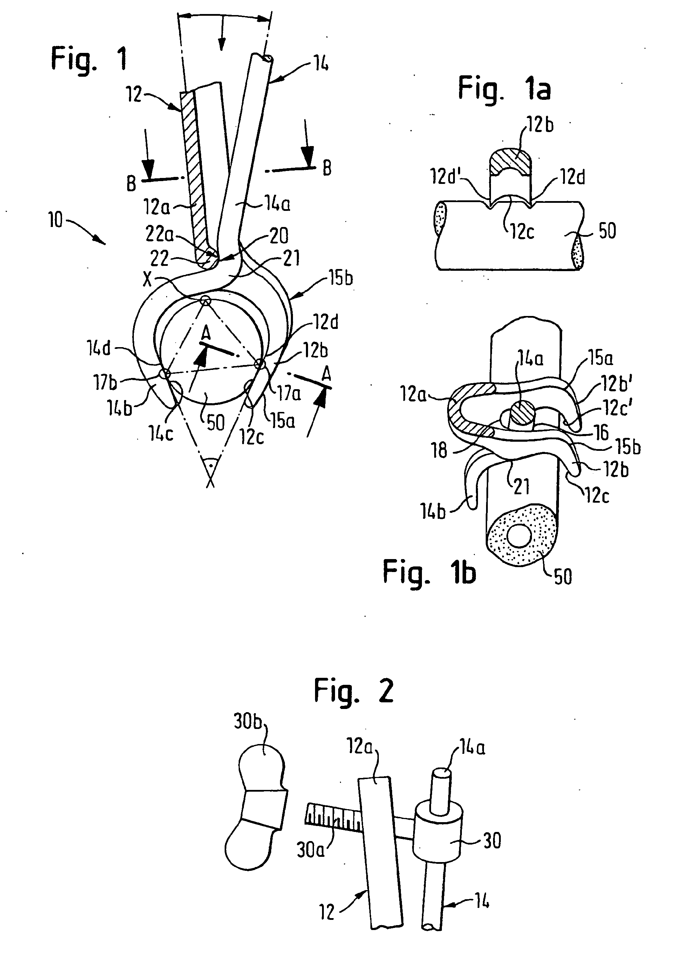

A preferred embodiment of the present invention as shown in Figure 1 is designated as a whole by the reference number 10 and in the following will be called "pliers 10". The pliers 10 consist of a first lever element 12 and a second lever element 14 which are assembled to form the pliers 10. A surgeon's hand would grip the pliers at a first 12a and second 14a handling portions 14a of the first 12 and the second 14 lever element. A bone 50 can be gripped or clamped by means of a first gripping portion 14b and a second gripping portion 12b, 12b'.

When introducing the elements of the pliers 10 into the surgical field in a patient's body, a surgeon would first introduce the second lever element 14 and then the first lever element 12, such that the knee portion 21 approaches getting close to the guide surface 20. Said knee portion 21 can be introduced through a guide surface between two fork-like or prong-like extensions 15a, 15b, to form a secure connection between the first lever element 12 and the second lever element 14. At the end of the guide surface between the prong-shaped extensions 15a, 15b of the second gripping portion 12b, 12b', a sliding surface 20a is provided which touches the guide surface 20 once assembled, such that the two lever elements 12, 14 can be rotated to urge areas of the first and second gripping portions 12b, 12b, 12b' against corresponding attachment points or attachment areas 17a, 17b with respect to the bone 50. To avoid a rotational movement of the levers and thus of the pliers 10 as a whole, the pliers are preferably attached to the bone 50, such that the pliers touch the bone at an additional support point or area X.

The first and second gripping portions 14b, 12b, 12b' are provided with corresponding first and second gripping surfaces 14c, 12c, 12c'.

As shown in particular in Figure 1a, the first and second gripping portions 14b, 12b, 12b' are provided with the gripping surface 12c which can have a ridged or spiked structure or the like. Furthermore, extended edges 12d, 12d' can be provided at the edges of the corresponding gripping surfaces. The extended edges 12d, 12d' can be urged into the surface of the bone 50, such that the attachment of the pliers 10 to the bone can be made more secure against being displaced by a torque force or shifting force acting on the pliers 10.

As can be seen in Figure 1, the gripping area of the pliers 10 touches the bone 50 at three different points or areas X, 17a, 17b and thus additionally helps to avoid the pliers 10 being displaced, once fixed to a particular location on the bone.

Figure 1b shows the situation immediately after inserting and assembling the levers and during the closing operation for urging the gripping surfaces 14c, 12c, 12c' against the surface of the bone 50.

In accordance with Figure 2, the first lever element 12 can be provided with a U-shaped recess at its upper end. A securing element 30, and in particular a bushing or cylindrical bushing 30, the shape of which depends of course on the cross-sectional shape of the second lever element 14, has been shifted over the upper end of the second lever element 14. A protrusion 30a - in the present case a threaded shaft - is fixed to the bushing 30. The threaded shaft extends through the U-shaped recess (not shown) in the upper end of the first lever element 12. A thumb nut is used as a fixing element 30b, to urge the first handling portion 12a in a rotational movement of the lever 12 towards the second handling portion 14a of the second lever element 14. Once tightened, the thumb nut 30b will of course securely hold the lever elements 12, 14 in a particular position.

Accordingly, the fixing device or pliers 10 according to the present invention exhibit the advantage that is only necessary to provide a small soft tissue portal in a patient, since it is possible to introduce the lever elements 12, 14 separately and to assemble the lever elements 12, 14 in the surgical field within the patient's body. Furthermore, since according to this embodiment of the invention, it is possible to securely fix the pliers 10 to a bone without penetrating the bone tissue, the injury to the bone is very minor and the fixing device 10 according to the invention allows other surgical instruments, implants or the like to be introduced, since the pliers 10 do not occupy much space within the bone.

### List of reference numerals

- 10: fixing device, pliers
- 12: first lever element
- 12a: first handling portion
- 12b, 12b': first gripping portion
- 12c, 12c': first gripping surface
- 12d: extended edge
- 14: second lever element
- 14a: second handling portion
- 14b: second gripping portion
- 14c: second gripping surface
- 14d: extended edge
- 15a, 15b: prong-shaped extension
- 16: insertion section
- 17a, 17b: attachment points or attachment areas
- 18: receiving section
- 20: guide surface
- 20a: sliding surface
- 21: knee portion
- 22: joint section
- 22a: sliding surface
- 30: securing element, bushing, cylindrical bushing
- 30a: protrusion, e.g. threaded shaft
- 30b: fixing element, e.g. thumb nut
- 50: bone
- X: support point or area

## Claims

1. A fixing device (10) for securely fixing surgical equipment, for instance a reference element or reference array, to a surgical field of a human or animal body, in particular a bone (50), wherein said device comprises:
- a first lever element (12) with a first gripping portion (12b) and a first handling portion (12a);
- a second lever element (14) with a second gripping portion (14b) and a second handling portion (14a);
- wherein said first and second lever elements (12, 14) can be connected such that they pivoted and detached with respect to each other,
and wherein one of the first and second lever elements (12, 14) integrally includes a guide surface (20) for allowing a joint section (22) to pivot or rotate, said joint section (22) having a sliding surface (22a) and being integrally formed with the other of the first and second lever elements (12, 14),
**characterized in that** said first and second lever elements (12, 14) are detachable with respect to each other during use of the fixing device and **in that** the other of the first and second lever elements includes said sliding surface between two prong-like or fork-like extensions of the gripping portion.

2. The fixing device according to claim 1, wherein one of the first and second lever elements (12, 14) comprises a receiving section (18) and the other of the first and second lever elements (12, 14) comprises an insertion section (16), such that said insertion section (16) can be removed and is received in said receiving section (18) such that it can be pivoted.

3. The fixing device according to anyone of claims 1 or 2, further comprising a securing element (30) for securely maintaining an angle between the lever elements (12, 14) for securely urging said gripping portions (12b, 12b', 14b) against a bone (50).

4. The fixing device according to anyone of claims 2 or 3, wherein the receiving section (18) has a hole-like, groove-like or slit-like shape for guiding said insertion section (16) into an operating position within the receiving section (18).

5. The fixing device according to anyone of claims 1 to 4, wherein each of said first and second gripping portions (14b 12b, 12b') have at least one gripping surface (14c, 12c, 12c').

6. The fixing device according to anyone of claims 1 to 5, wherein the first and second lever elements (12, 14) are formed to provide a plier-like instrument (10), once introduced.

7. The fixing device according to anyone of claims 5 or 6, wherein the gripping surfaces (12c, 14c, 14c') are broadened in a direction perpendicular to the main extension of the corresponding lever element (12, 14) and/or are provided with at least one, preferably two extended edges (14d, 12d, 12d') directed towards the opposite gripping surface.

## Patentansprüche

1. Befestigungsvorrichtung (10) zum sicheren Befestigen eines chirurgischen Geräts, beispielsweise eines Referenzelements oder einer Referenzanordnung, an einem chirurgischen Gebiet eines menschlichen oder tierischen Körpers, insbesondere an einem Knochen (50), wobei die Vorrichtung umfasst:
- ein erstes Hebelelement (12) mit einem ersten Greifabschnitt (12b) und einem ersten Handhabungsabschnitt (12a);
- ein zweites Hebelelement (14) mit einem zweiten Greifabschnitt (14b) und einem zweiten Handhabungsabschnitt (14a);
- wobei das erste und das zweite Hebelelement (12, 14) in der Weise miteinander verbunden werden können, dass sie relativ zueinander geschwenkt und voneinander gelöst werden können, und
- wobei das erste oder das zweite Hebelelement (12, 14) einteilig eine Führungsoberfläche (20) aufweist, um einem Gelenkabschnitt (20) zu ermöglichen, zu schwenken oder sich zu drehen, wobei der Gelenkabschnitt (22) eine Gleitoberfläche (22a) besitzt und mit dem jeweils anderen des ersten und des zweiten Hebelelements (12, 14) einteilig ausgebildet ist,
**dadurch gekennzeichnet, dass** das erste und das zweite Hebelelement (12, 14) während der Verwendung der Befestigungsvorrichtung voneinander lösbar sind und dass das jeweils andere des ersten und des zweiten Hebelelements zwischen zwei zinkenartigen oder gabelartigen Verlängerungen des Greifabschnitts eine Gleitoberfläche aufweist.

2. Befestigungsvorrichtung nach Anspruch 1, wobei das erste oder das zweite Hebelelement (12, 14) einen Aufnahmeabschnitt (18) umfasst und das jeweils andere des ersten und des zweiten Hebelelements (12, 14) einen Einsteckabschnitt (16) umfasst, derart, dass der Einsteckabschnitt (16) aus dem Aufnahmeabschnitt (18) entnommen und in ihm aufgenommen werden kann, so dass er geschwenkt werden kann.

3. Befestigungsvorrichtung nach einem der Ansprüche 1 oder 2, die ferner ein Sicherungselement (30) umfasst, um einen Winkel zwischen den Hebelelementen (12, 14) sicher aufrechtzuerhalten, um die Greifabschnitte (12b, 12b', 14') sicher gegen einen Knochen (50) zu drängen.

4. Befestigungsvorrichtung nach einem der Ansprüche 2 oder 3, wobei der Aufnahmeabschnitt (18) eine lochartige, nutartige oder schlitzartige Form hat, um den Einsteckabschnitt (16) in eine Betriebsposition in dem Aufnahmeabschnitt (18) zu führen.

5. Befestigungsvorrichtung nach einem der Ansprüche 1 bis 4, wobei der erste und der zweite Greifabschnitt (14b, 12b, 12b') wenigstens eine Greifoberfläche (14c, 12c, 12c') haben.

6. Befestigungsvorrichtung nach einem der Ansprüche 1 bis 5, wobei das erste und das zweite Hebelelement (12, 14) ausgebildet sind, um, sobald sie eingeführt sind, ein zangenartiges Instrument (10) zu schaffen.

7. Befestigungsvorrichtung nach einem der Ansprüche 5 oder 6, wobei die Greifoberflächen (12c, 14c, 14c') in einer Richtung senkrecht zu der Haupterstreckung des entsprechenden Hebelelements (12, 14) erweitert sind und/oder mit wenigstens einer, vorzugsweise mit zwei verlängerten Kanten (14d, 12d, 12d') versehen sind, die zu der gegenüberliegenden Greifoberfläche gerichtet sind.

## Revendications

1. Dispositif de fixation (10) pour fixer fermement un équipement chirurgical, par exemple un élément de référence ou un groupement de référence, à un champ opératoire d'un corps humain ou animal, en particulier un os (50), dans lequel ledit dispositif comprend :
- un premier élément de levier avec une première partie d'agrippement (12b) et une première partie de manipulation (12a) ;
- un deuxième élément de levier (14) avec une deuxième partie d'agrippement (14b) et une deuxième partie de manipulation (14a) ;
- dans lequel lesdits premier et deuxième éléments de levier (12, 14) peuvent être reliés de telle sorte qu'on puisse les faire pivoter et qu'on puisse les détacher l'un par rapport à l'autre
- et dans lequel l'un des premier et deuxième éléments de levier (12, 14) comprend de façon intégrée une surface de guidage (20) pour permettre à une section de raccord (22) de pivoter ou de tourner, ladite section de raccord (22) comportant une surface de coulissement (22a) et étant formée d'un seul tenant avec l'autre des premier et deuxième éléments de levier (12,14),
**caractérisé en ce que** lesdits premier et deuxième éléments de levier (12, 14) sont détachables l'un de l'autre durant l'utilisation du dispositif de fixation,
l'autre des premier et deuxième éléments de levier comprenant ladite surface de coulissement entre des prolongements en forme de fourche ou en forme de griffe de la partie d'agrippement.

2. Dispositif de fixation selon la revendication 1, dans lequel l'un des premier et deuxième éléments de levier (12, 14) comprend une section de réception (18) et l'autre des premier et deuxième éléments de levier (12, 14) comprend une section d'insertion (16), de telle sorte que ladite section d'insertion (16) puisse être retirée et soit reçue dans ladite section de réception (18) de telle sorte qu'on puisse la faire pivoter.

3. Dispositif de fixation selon l'une quelconque des revendications 1 ou 2, comprenant de plus un élément de maintien (30) pour maintenir fermement un angle entre les éléments de levier (12, 14) pour pousser fermement lesdites parties d'agrippement (12b, 12b', 14b) contre un os (50).

4. Dispositif de fixation selon l'une quelconque des revendications 2 ou 3, dans lequel la section de réception (18) comporte une forme en trou, en rainure ou en fente pour guider ladite section d'insertion (16) dans une position opérationnelle à l'intérieur de la section de réception (18).

5. Dispositif de fixation selon l'une quelconque des revendications 1 à 4, dans lequel chacune desdites première et deuxième parties d'agrippement (14b, 12b, 12b') comporte au moins une surface d'agrippement (14c, 12c, 12c').

6. Dispositif de fixation selon l'une quelconque des revendications 1 à 5, dans lequel les premier et deuxième éléments de levier (12, 14) sont formés de façon à constituer un instrument en forme de pince (10), une fois introduits.

7. Dispositif de fixation selon l'une quelconque des revendications 5 ou 6, dans lequel les surfaces d'agrippement (12c, 14c, 14c') sont élargies dans une direction perpendiculaire à l'étendue principale de l'élément de levier correspondant (12, 14) et/ou sont munies d'au moins un, et de préférence de deux, bords étendus (14d, 12d, 12d') dirigés vers la surface d'agrippement opposée.
